# FASCICULE DE BREVET EUROPEEN

(11) **EP 0 962 235 B1**
(45) Date de publication et mention de la délivrance du brevet: **19.05.2004**
(21) Numéro de dépôt: 99401361.3
(22) Date de dépôt: 04.06.1999
(51) Int. Cl.: A61N 1/362

(54) **Dispositif médical implantable actif à gestion perfectionnée des périodes réfractaires**
Implantierbare medizinische Vorrichtung mit verbesserter Refraktärzeitsverwaltung
Implantable medical device with improved management of refractory periods

(30) Priorité: 05.06.1998 FR 9807055
(43) Date de publication de la demande: 08.12.1999
(73) Titulaire: ELA MEDICAL (Société anonyme), 92541 Montrouge (FR)
(72) Inventeur: Legay, Thierry, 91640 Fontenay les Briis (FR); Bonnet, Jean-Luc, 92120 Montrouge (FR); Bouhour, Anne, 92410 Ville d'Avray (FR)
(74) Mandataire: Dupuis-Latour, Dominique

(56) Documents cités:
- EP-A- 0 318 304
- EP-A- 0 562 237
- US-A- 4 779 617
- US-A- 4 974 589
- US-A- 5 388 586
- US-A- 5 540 725

## Description

L'invention concerne les "dispositifs médicaux implantables actifs" tels que définis par la directive 90/385/CEE du 20 juin 1990 du Conseil des communautés européennes, et plus précisément les dispositifs stimulateurs cardiaques (pour le traitement des bradycardies), défibrillateurs et/ou cardioverteurs (pour les traitement des tachycardies), ainsi que les resynchroniseurs ou "dispositifs multisites" (pour le traitement des désynchronisations entre cavités).

Tous ces dispositifs comportent des moyens de détection d'activité, c'est-à-dire de détection des dépolarisations spontanées du myocarde, ainsi des moyens de stimulation de ce myocarde.

Pendant et après chaque activation des moyens de stimulation, il est prévu une période dite "période réfractaire" pendant laquelle est opérée une déconnexion ou "blanking" des circuits de détection, de manière à masquer les perturbations des amplificateurs juste après la stimulation (perturbations dues à l'écoulement des charges à l'interface électrode/ myocarde).

Une période réfractaire longue permet de prendre une bonne marge de sécurité pour l'élimination de ces perturbations. Mais elle présente l'inconvénient de masquer l'écoute des signaux émis par le myocarde pendant une période de temps importante.

Or il est important de pouvoir écouter le rythme spontané du patient le plus tôt possible après la stimulation, afin de détecter le plus précocement possible une onde de dépolarisation révélatrice d'une activité spontanée des cellules myocardiques et permettre ainsi d'exécuter par exemple des algorithmes très précis de contrôle du rythme cardiaque donnant lieu à un comportement plus physiologique de la prothèse, ou encore permettre une réduction de la consommation en ne délivrant que des stimulations appropriées. Cette détection est également utilisée pour commander le fonctionnement de certains algorithmes tels que les algorithmes de repli ou de lissage, etc. D'autre part, la détection du rythme ventriculaire spontané, notamment l'analyse de sa stabilité, est dans certains défibrillateurs implantables un paramètre essentiel de déclenchement de la thérapie de choc.

Pour tenir compte des différentes situations possibles, la période réfractaire est généralement variable, avec une partie fixe ou préprogrammée dite "période réfractaire absolue" (PRA) et une partie variable dite "période réfractaire relative" (PRR).

La durée de la PRR peut être adaptée en fonction de divers paramètres.

On peut ainsi faire varier cette PRR en fonction de l'amplitude de stimulation programmée, car la perturbation dont on veut s'affranchir dure moins longtemps dans le cas d'une stimulation à faible énergie (par exemple 1,5 V) que dans celui d'une stimulation à plus forte énergie (par exemple 5 V).

Une autre technique, proposée par exemple par le US-A-4 974 589 et également utilisée dans les stimulateurs de type *Chorus* d'Ela Médical, consiste à détecter en cours de PRA la présence d'un potentiel résiduel en sortie des amplificateurs d'entrée des circuits de détection du signal cardiaque endocavitaire. Si un potentiel résiduel est détecté, on prolonge alors la PRA d'une durée prédéterminée, ci-après désignée "période élémentaire", et cette période élémentaire est réitérée (ou "recyclée" ou "redéclenchée") tant qu'un potentiel résiduel post-stimulation apparaît en sortie des amplificateurs de détection. Quand le potentiel résiduel détecté est inférieur à un seuil donné, le recyclage automatique des périodes élémentaires cesse et le système passe en mode d'écoute des signaux cardiaques.

La présente invention vise un perfectionnement aux dispositifs médicaux de ce type, perfectionnement qui permette une gestion très fine de la durée de la PRR de manière à réduire celle-ci au strict minimum et maximiser ainsi la durée d'écoute du rythme spontané du patient, ceci pour pouvoir exécuter de façon optimale les divers algorithmes de contrôle du dispositif

Le dispositif de l'invention est du type général décrit dans le US-A-4 974 589, c'est-à-dire un dispositif dans lequel il est prévu des moyens pour appliquer une période réfractaire aux moyens de détection après activation des moyens de stimulation, cette période réfractaire comprenant une période réfractaire absolue, fixe ou préprogrammée, et une période réfractaire relative, variable, la période réfractaire relative comprenant une succession de périodes élémentaires de durée fixe ou programmable réitérées jusqu'à écoulement d'une période élémentaire sans occurrence d'un potentiel résiduel de niveau supérieur à un seuil donné en sortie des moyens de détection.

Selon l'invention, les moyens pour appliquer la période réfractaire : a) produisent, sur chaque réitération d'une période élémentaire, une succession de sous-périodes de durée chacune inférieure à celle d'une période élémentaire ; b) détectent l'occurence éventuelle d'un potentiel pendant chaque sous-période ; et c1) en cas d'occurence, réitèrent la période élémentaire dès la fin de la sous-période où l'occurence a été détectée ; ou c2) en l'absence d'occurence, réitèrent si nécessaire la sous-période jusqu'à la fin de la période élémentaire.

La durée de la sous-période est avantageusement un sous-multiple de la durée de la période élémentaire, et le ratio entre la durée de la sous-période et celle de la période élémentaire est d'au moins 2 : 1, de préférence au moins 6 : 1.

On va maintenant décrire un exemple de mise en oeuvre de l'invention, en référence à la figure unique annexée, qui représente deux chronogrammes montrant la décomposition d'une période réfractaire, respectivement selon l'art antérieur et selon l'invention.

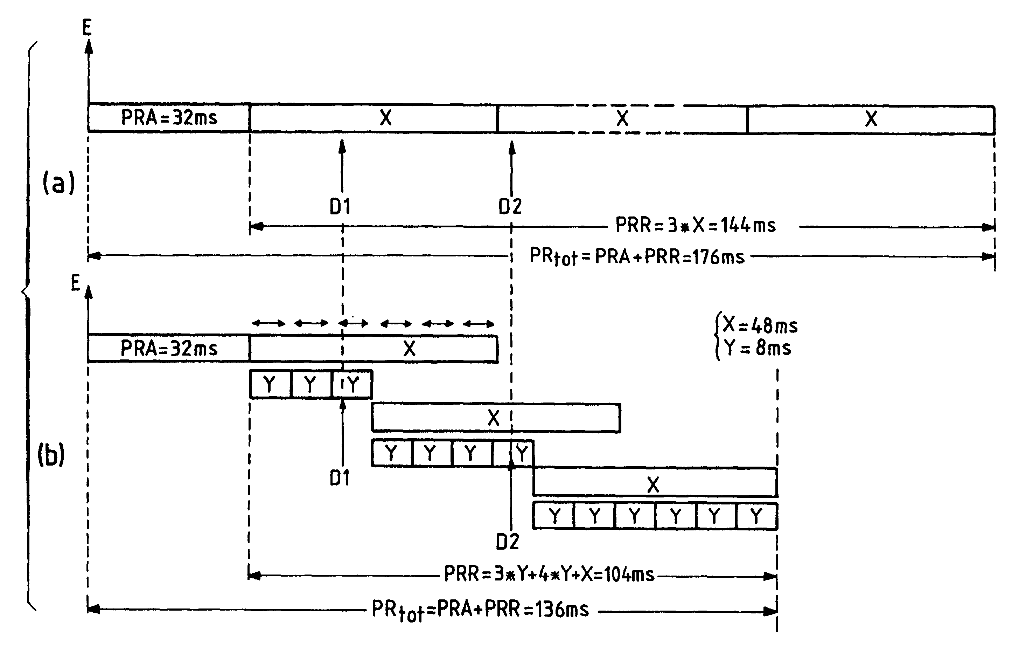

La figure 1(a) correspond à une période réfractaire telle que définie par un dispositif de type connu, par exemple un stimulateur cardiaque *Chorus* d'Ela Médical : juste après un événement cardiaque E correspondant à une stimulation, le dispositif applique une période réfractaire absolue PRA, typiquement de 32 ms, au cours de laquelle aucune détection ne peut affecter le comportement de la prothèse cardiaque.

La période réfractaire se poursuit par une période réfractaire relative PRR au cours de laquelle les détections qui se produisent n'ont pas d'influence sur le comportement des algorithmes de la prothèse, mais provoquent un redéclenchement de la PRR pour une durée X, typiquement X = 48 ms, jusqu'à ce qu'une période élémentaire complète se soit déroulée sans qu'aucune détection ne soit intervenue.

Ainsi, dans l'exemple illustré, une détection D1 intervenue au cours de la première période élémentaire X provoque le redéclenchement de celle-ci, et donc la prolongation de la PRR d'une nouvelle durée X. Une deuxième détection D2 survenant au cours de cette deuxième période élémentaire, un nouveau redéclenchement intervient, pour une troisième période élémentaire X. Aucune détection n'étant intervenue au cours de la troisième période élémentaire X, la prothèse passe ensuite en mode d'écoute des signaux cardiaques spontanés.

La période réfractaire relative est donc, dans cet exemple, PRR = 3_{*}X = 144 ms, soit une durée totale PRₜₒₜ = PRA + PRR = 32 + 144 = 176 ms pour la période réfractaire.

On notera que, dans l'exemple illustré, la deuxième détection (D2) est intervenue à un moment relativement précoce de la deuxième période élémentaire X, de sorte qu'il s'est écoulé une durée presque égale à 2_{*}X entre la dernière détection d'un potentiel résiduel (D2) et le début de la phase d'écoute des signaux cardiaques.

L'invention propose de perfectionner le mode de détermination de la PRR de manière à écourter ce délai et permettre de passer plus tôt en mode d'écoute des signaux cardiaques.

Pour ce faire, comme illustré figure 1(b), après la PRA une première période élémentaire X est déclenchée pour initier la PRR.

Mais dans ce cas le redéclenchement de la période élémentaire X est opérée de manière différente : après avoir déclenché la période élémentaire X, le dispositif définit une succession de sous-périodes Y et examine si, au cours de chacune des ces sous-périodes, un potentiel résiduel est détecté ou non.

La durée Y est choisie très inférieure à la durée X, de préférence Y sous-multiple de X, par exemple Y = X / 6 = 8 ms.

Si une détection intervient au cours de l'une de ces sous-périodes Y (par exemple la détection D1 au cours de la troisième sous-période Y de la première période élémentaire X), alors la période élémentaire X est redéclenchée dès la fin de cette sous-période, sans attendre la fin de la période élémentaire en cours.

Ainsi, dans l'exemple illustré sur la figure, la deuxième période élémentaire X est déclenchée, 3*Y = 24 ms après le début de la PRR, au lieu de l'être 48 ms après, comme avec la technique connue correspondant à la figure 1(a).

Le même processus de détection/redéclenchement est mis en oeuvre au cours de la deuxième période élémentaire X : ainsi, si une détection D2 intervient au cours de la quatrième sous-période Y, la période élémentaire X est redéclenchée dès la fin de cette quatrième sous-période, et ainsi de suite.

La PRR prend fin lorsque s'est écoulée une période élémentaire X complète (ou, en d'autres termes, six sous-périodes Y consécutives) sans qu'aucun potentiel résiduel n'ait été détecté.

Dans l'exemple illustré, la PRR prend ainsi fin au bout de 3_{*}Y + 4_{*}Y + X = 104 ms, soit une durée totale PRₜₒₜ = PRA + PRR = 32 + 104 = 136 ms pour la période réfractaire.

On voit ainsi que, pour des détections D1 et D2 survenant au même instant, dans cet exemple particulier on a, grâce à la technique de l'invention, réduit la durée de la PRR de 40 ms, soit de 28 %, permettant ainsi une écoute beaucoup plus précoce des signaux spontanés du coeur.

Les valeurs de la PRA, de X et de Y sont de préférence programmables pour une prothèse donnée, ou même pour un mode de fonctionnement particulier d'une prothèse, et éventuellement (mais non nécessairement) programmables par le médecin.

Si le dispositif concerne une pluralité de cavités, la technique de l'invention résout tous les cas de protection de façon générale, et il n'est pas nécessaire de prévoir des protections spécifiques de type "blanking logique ventriculaire post-stimulation auriculaire". En effet, le dernier événement apparu en période d'écoute dans une quelconque cavité cardiaque pourra recycler la génération des périodes élémentaires des PRR dans toutes les cavités cardiaques possédant un système propre de détection. Dans tous les cas, la durée finale de la période réfractaire, pour chaque cavité détectée indépendamment, sera toujours adéquate et minimale puisque basée sur la présence ou non de potentiels parasites en sortie de sa propre chaîne de détection. En d'autres termes, la durée de la PRR pour une cavité particulière ne dépend que de l'accumulation dans cette cavité des potentiels post-stimulation créés par l'ensemble des événements stimulés/détectés se produisant dans l'ensemble du coeur.

Cette technique permet également de prendre en compte toutes les perturbations électromagnétiques, internes ou externes, touchant la cavité considérée. En effet, le redéclenchement persiste en présence de bruits électriques, maintenant la prothèse dans un état réfractaire tant que les perturbations sont présentes.

A cet égard, les paramètres X et Y définissant la PRR peuvent être programmés pour rendre la prothèse réfractaire à des bruits dont la fréquence excède 20 Hz, comme spécifié par la norme EN 45 502. Par exemple, avec une période élémentaire X = 48 ms redéclenchable toutes les Y = 8 ms, une prothèse passera en mode asynchrone s'il apparaît un bruit persistant de fréquence supérieure à 20,8 Hz.

On notera enfin que le système selon l'invention peut être aisément mis en oeuvre par un circuit en logique câblée, ce qui permet d'abaisser la consommation du dispositif par rapport à une mise en oeuvre logicielle par microprocesseur.

## Revendications

1. Un dispositif médical implantable actif, notamment un dispositif stimulateur, défibrillateur, cardioverteur et/ou resynchroniseur cardiaque, comprenant des moyens de détection d'activité du myocarde et des moyens de stimulation de ce myocarde,
dans lequel il est prévu des moyens pour appliquer une période réfractaire aux moyens de détection après activation (E) des moyens de stimulation, cette période réfractaire (PRₜₒₜ) comprenant une période réfractaire absolue (PRA), fixe ou préprogrammée, et une période réfractaire relative (PRR), variable,
la période réfractaire relative comprenant une succession de périodes élémentaires (X) de durée fixe ou programmable réitérées jusqu'à écoulement d'une période élémentaire sans occurrence d'un potentiel résiduel de niveau supérieur à un seuil donné en sortie des moyens de détection,
dispositif **caractérisé en ce que** les moyens pour appliquer la période réfractaire :
a) produisent, sur chaque réitération d'une période élémentaire, une succession de sous-périodes (Y) de durée chacune inférieure à celle d'une période élémentaire,
b) détectent l'occurence éventuelle d'un potentiel (D1, D2) pendant chaque sous-période, et
c1) en cas d'occurence, réitèrent la période élémentaire dès la fin de la sous-période où l'occurence a été détectée, ou
c2) en l'absence d'occurence, réitèrent si nécessaire la sous-période jusqu'à la fin de la période élémentaire.

2. Le dispositif de la revendication 1, dans lequel la durée de la sous-période est un sous-multiple de la durée de la période élémentaire.

3. Le dispositif de la revendication 1, dans lequel le ratio entre la durée de la sous-période et celle de la période élémentaire est d'au moins 2 : 1, de préférence au moins 6 : 1.

## Patentansprüche

1. Aktive implantierbare medizinische Vorrichtung, insbesondere ein Herzschrittmacher, Defibrillator, Kardioverter und/oder Herz-Resynchronisierer, umfassend Mittel zur Erfassung der Herzmuskel-Aktivität und Mittel zur Stimulation dieses Herzmuskels,
in welchem Mittel vorgesehen sind zum Anwenden einer Refraktärzeit auf die Erfassungsmittel nach Aktivierung (E) der Stimulationsmittel, wobei diese Refraktärzeit (PRₜₒₜ) eine absolute, feste und vorprogrammierte Refraktärzeit (PRA), und eine relative, variable Refraktärzeit (PRR) umfassen,
wobei die relative Refraktärzeit eine Folge von Elementarperioden (X) von fester oder programmierbarer Dauer umfasst, die wiederholt werden bis Ablauf einer Elementarperiode ohne Auftreten eines Restpotentials am Ausgang der Erfassungsmittel, mit einem Niveau, das höher ist als ein gegebener Schwellwert,
wobei die Vorrichtung **dadurch gekennzeichnet ist, dass** die Mittel zur Anwendung der Refraktärperiode:
a) bei jeder Wiederholung einer Elementarperiode eine Reihe von Unterperioden (Y) produzieren, deren Dauer jeweils geringer ist als die einer Elementarperiode,
b) das etwaige Auftreten eine Potentials (D1, D2) während jeder Unterperiode erfassen, und
c1) im Fall des Auftretens die Elementarperiode seit dem Ende der Unterperiode wiederholen, in der das Auftreten erfasst wurde, oder
c2) bei Abwesenheit des Auftretens, die Unterperiode, wenn notwendig, bis zu dem Ende der Elementarperiode wiederholen.

2. Vorrichtung gemäß Anspruch 1, in welcher die Dauer der Unterperiode ein Bruchteil der Dauer der Elementarperiode ist.

3. Vorrichtung gemäß Anspruch 1, in welchem das Verhältnis zwischen der Dauer der Unterperiode und derjenigen der Elementarperiode mindestens 2:1 beträgt, vorzugsweise mindestens 6:1.

## Claims

1. An active implantable medical device, in particular a pacemaker, defibrillator, cardioverter and/or cardiac resynchronizer device, comprising means for detecting the activity of the myocardium and means for stimulating said myocardium,
wherein there is provided means for applying a refractory period to the detection means after activation (E) of the stimulation means, said refractory period (PRₜₒₜ) including an absolute refractory period (PRA), which is fixed or pre-programmed, and a relative refractory period (PRR), which is variable,
the relative refractory period comprising a sequence of elementary periods (X) having a fixed or programmable duration and which are retriggered until the lapse of an elementary period without occurrence on the output of the detection means of a residual potential with a level higher than a given threshold,
said device being **characterised in that** the means for applying the refractory period:
a) produces, on each retriggering of an elementary period, a sequence of sub-periods (Y) having each a duration shorter than the duration of an elementary period,
b) detects the possible occurrence of a potential (D1, D2) during every sub-period, and
c1) in case of an occurrence, retriggers the elementary period just at the end of the sub-period during which the occurrence was detected, and
c2) in the absence of an occurrence, retriggers if necessary the sub-period until the end of the elementary period.

2. The device of claim 1, wherein the duration of each sub-period is a submultiple of the duration of the elementary period.

3. The device of claim 1, wherein the ratio of the duration of the sub-period to the duration of the elementary period is at least 2:1, preferably at least 6:1.
